# EUROPEAN PATENT APPLICATION

(11) **EP 4 813 285 A1**
(43) Date of publication of application: **30.09.2026**
(21) Application number: 25208934.7
(22) Date of filing: 15.10.2025
(51) Int. Cl.: A61B 5/00, A61B 5/024

(54) **DEVICE FOR MONITORING PHYSIOLOGICAL, CARDIOVASCULAR AND BRAIN HEALTH PARAMETERS OF USERS AND METHOD THEREOF**

(30) Priority: 28.03.2025 IN 202541029790
(71) Applicant: Ultrahuman Healthcare Private Limited, Bangalore South, Karnataka 560068 (IN)
(72) Inventor: NAMDEO, Yogansh, 560100 Karnataka (IN); JAYAN, Anoop, 560058 Karnataka (IN); PRADEEP, Vishnu, 560099 Karnataka (IN); JOSHI, Akshay, 413304 Maharashtra (IN); MALLAPPA, Yogesh, 638461 Tamil Nadu (IN)
(74) Representative: Office Freylinger

(57) **Abstract**

The present disclosure relates to a device [100] for monitoring physiological, cardiovascular and brain health parameters of users and a method thereof. The device [100] comprises a data collection unit [102], a first trained model [104] and a transmission unit [106] connected to each other. The data collection unit [102], comprising at least a set of sensors and is configured to receive a set of user data. Further, the first trained model [104] is configured to: integrate, one or more target user data from the set of user data based on predefined data integration technique(s); generate, an enhanced dataset based on the integrated one or more target user data; and detect, abnormalities associated with the user based on at least the enhanced dataset. Furthermore, the transmission unit [106] is configured to transmit, on a user device, a set of notification associated with the detected abnormalities.

## Description

### TECHNICAL FIELD

The present disclosure generally relates to the field of health and fitness monitoring techniques. Particularly, the present disclosure relates to the field of sensor-based devices for monitoring health and fitness of users. More particularly, the present disclosure relates to a non-invasive devices for monitoring physiological, cardiovascular and brain health parameters of the user.

### BACKGROUND

The following description of the related art is intended to provide background information pertaining to the field of the disclosure. This section may include certain aspects of the art that may be related to various features of the present disclosure. However, it should be appreciated that this section is used only to enhance the understanding of the reader with respect to the present disclosure, and not as admissions of the prior art.

A wearable device is an electronic device that is designed to be used while being worn by a user of such electronic device. In today's digital age, one or more types of wearable devices are available such as, but not limited to, smart rings, smartwatches, fitness trackers and wrist bands. Further, these wearable devices include a set of sensors such as, but not limited to, an accelerometer, a gyroscope and photoplethysmography (PPG) sensors. The said set of sensors are responsible for measuring a set of data related to the health of the user of a wearable device. The set of data may include heart rate, oxygen saturation, respiration rate, blood pressure, etc. of the user of the wearable device. However, the existing wearable device are only capable of being worn in particular region/area of the body i.e., on limbs of the user. Further, the existing wearable devices struggle with the accuracy in providing data related to areas like neck area and/or high blood flow areas of the user body, because the existing wearable devices are applied in the areas with less perfusion and excessive movements. Further, it is to be understood that accurate measurement of the health related data from such other areas is important, for assessing in real time the cardiovascular health, hydration levels, and respiratory efficiency of the user of the wearable device.

Further, another shortcoming in the existing wearable devices for monitoring physiological, cardiovascular and health parameters of a user mostly rely on technologies, such as photoplethysmography (PPG) sensors to measure blood volume changes. However, in the existing solutions such as wearable devices with the PPG sensors struggles to accurately provide the set of details related to the health of the user as such wearable devices are worn in areas of the body with less perfusion or excessive movement such as wrist, fingers, etc. Also, in the existing wearable devices with the PPG sensors gets affected by movement artifacts and poor contact with the skin.

The existing solution also lacks the ability to provide high-resolution data in real-time. Further, the assessment of hydration levels using far infrared (FIR) technology is underutilized and often inaccurate due to inconsistent placement of the wearable device. Also, the methods for measuring blood flow in the existing solution are indirect and not optimized to efficiently track real-time blood flow from region around the neck of the user. Further, traditional pulse oximeters, which measure oxygen saturation (SpO2), are configured to perform well on particular regions of the body of the user, such as the fingertip or earlobe, but are less reliable at alternative locations, such as the neck. Furthermore, the existing solutions are also inaccurate in monitoring the respiratory rate due to body part movement performed by the user and therefore such wearable devices require additional sensors to accurately monitor the respiratory rate.

Therefore, there are a number of limitations to the existing solutions and in order to overcome these and such other limitations of the known solutions it is necessary to provide a non-invasive device optimized for placement on any particular region/area on the body of the user such as near jugular veins of a user to monitor physiological, cardiovascular and brain health parameters of the user.

### SUMMARY OF DISCLOSURE

This section is provided to introduce certain aspects of the present disclosure in a simplified form that are further described below in the detailed description. This summary is not intended to identify the key features or the scope of the claimed subject matter.

An aspect of the present disclosure relates to a device for monitoring physiological, cardiovascular and brain health parameters of a user. The device comprises a data collection unit, a first trained model connected to at least the data collection unit and a transmission unit connected to at least the first trained model. The data collection unit, comprising at least a set of sensors, is configured to receive a set of user data associated with a user. Further, the first trained model is configured to integrate, one or more target user data from the set of user data based on one or more predefined data integration technique. The first trained model is further configured to generate, an enhanced dataset based on the integrated one or more target user data, Further, the first trained model is configured to detect, one or more abnormalities associated with the user based on at least the enhanced dataset. Furthermore, the transmission unit is configured to transmit, on a user device, a set of notification associated with the detected one or more abnormalities.

In exemplary aspect of the present disclosure, the device is configured to receive via the set of sensors, the set of user data from a target body location of the user.

In exemplary aspect of the present disclosure, the target body location is determined based on a set of body location determination rules.

In exemplary aspect of the present disclosure, the set of body location determination rules is based on at least one of a jugular vein location and a high blood flow location of the user.

In exemplary aspect of the present disclosure, the set of sensors comprises at least one of a Photoplethysmography (PPG) sensor, and a Far Infrared (FIR) sensor.

In an exemplary aspect of the present disclosure, the set of user data comprises at least one of a real time physiological health data, a real time cardiovascular health data and a real time brain health data.

In an exemplary aspect of the present disclosure, the first trained model is further configured to: 1) fetch, from a database, a set of historical user data associated with the user; 2) determine, the one or more target user data based on comparing the set of user data and the set of historical user data; and 3) generate, the integrated one or more target user data based on the determined one or more target user data, and at least one predefined data integration technique from the one or more predefined data integration technique.

In an exemplary aspect of the present disclosure, the set of historical user data comprises at least one of a set of past physiological data, a set of past cardiovascular data and a set of past brain health data associated with the user.

In an exemplary aspect of the present disclosure, the one or more predefined data integration technique is at least one of a signal processing technique and a data processing technique.

In an exemplary aspect of the present disclosure, the first trained model is further configured to: 1) detect, a noise parameter associated with the integrated one or more target user data based on analyzing one or more of the set of user data, the set of historical user data and the integrated one or more target user data; and 2) filter, the noise parameter from the one or more target user data to generate the enhanced dataset.

In an exemplary aspect of the present disclosure, the noise parameter is generated based on at least one of a real time movement associated with the user and an activity performed by the user.

In an exemplary aspect of the present disclosure, the noise parameter is detected from the set of user data, the set of historical user data and the integrated one or more target user data based on a predefined noise detection technique.

In an exemplary aspect of the present disclosure, the one or more abnormalities is at least one of one or more physiological abnormalities, one or more cardiovascular abnormalities and one or more brain functions abnormalities associated with the user.

In an exemplary aspect of the present disclosure, the first trained model is further configured to: 1) fetch, a set of historical user data associated with the user of a predefined time period; and 2) determine, a key health parameter associated with the user based on comparing the set of user data and the set of historical user data, wherein the key health parameter indicates the one or more abnormalities.

In an exemplary aspect of the present disclosure, the key health parameter is determined in an event a value associated with one or more parameters of the enhanced dataset is above a predefined threshold value associated with said one or more parameters of the enhanced dataset.

Another aspect of the present disclosure relates to a method for monitoring physiological, cardiovascular and brain health parameters of a user. The method comprises receiving, at a data collection unit from a set of sensors, a set of user data associated with a user. Further, the method comprises integrating, by a first trained model, one or more target user data from the set of user data based on one or more predefined data integration technique. The method further comprises generating, by the first trained model, an enhanced dataset based on the integrated one or more target user data. Also, the method comprises detecting, by the first trained model, one or more abnormalities associated with the user based on at least the enhanced dataset. Furthermore, the method comprises transmitting, by a transmission unit, on a user device a set of notification associated with the detected one or more abnormalities.

In exemplary aspect of the present disclosure, the method further comprises receiving, via the set of sensors from a target body location of the user, the set of user data.

In exemplary aspect of the present disclosure, the target body location is determined based on a set of body location determination rules.

In exemplary aspect of the present disclosure, the set of body location determination rules is based at least one of a jugular vein location and a high blood flow location of the user.

In exemplary aspect of the present disclosure, the set of sensors comprises at least one of a Photoplethysmography (PPG) sensor, and a Far Infrared (FIR) sensor.

In an exemplary aspect of the present disclosure, the set of user data comprises at least one of a real time physiological health data, a real time cardiovascular health data and a real time brain health data.

In an exemplary aspect of the present disclosure, the method further comprises fetching, by the first trained model from a database, a set of historical user data associated with the user. Further, the method comprises determining, by the first trained model, the one or more target user data based on comparing the set of user data and the set of historical user data. The method further comprises generating, by the first trained model, the integrated one or more target user data based on the one or more target user data, and at least one predefined data integration technique from the one or more predefined data integration technique.

In an exemplary aspect of the present disclosure, the set of historical user data comprises at least one of a set of past physiological data, a set of past cardiovascular data and a set of past brain health data associated with the user.

In an exemplary aspect of the present disclosure, the one or more predefined data integration technique is at least one of a signal processing technique and a data processing technique.

In an exemplary aspect of the present disclosure, the method further comprises detecting, by the first trained model, a noise parameter associated with the integrated one or more target user data based on analyzing one or more of the set of user data, the set of historical user data and the integrated one or more target user data. Further, the method comprises filtering, the first trained model, the noise parameter from the one or more target user data to generate the enhanced dataset.

In an exemplary aspect of the present disclosure, the noise parameter is generated based on at least one of a real time movement associated with the user and an activity performed by the user.

In an exemplary aspect of the present disclosure, the noise parameter is detected from the set of user data, the set of historical user data and the integrated one or more target user data based on a predefined noise detection technique.

In an exemplary aspect of the present disclosure, the one or more abnormalities is at least one of one or more physiological abnormalities, one or more cardiovascular abnormalities and one or more brain functions abnormalities associated with the user.

In an exemplary aspect of the present disclosure, the method further comprises fetching, by the first trained model, a set of historical user data associated with the user of a predefined time period. Further, the method comprises determining, by the first trained model, a key health parameter associated with the user based on comparing the set of user data and the set of historical user data, wherein the key health parameter indicates the one or more abnormalities.

In an exemplary aspect of the present disclosure, the key health parameter is determined in an event a value associated with one or more parameters of the enhanced dataset is above a predefined threshold value associated with said one or more parameters of the enhanced dataset.

### OBJECTS OF DISCLOSURE

Some of the objects of the present disclosure which at least one embodiment disclosed herein satisfies are listed below.

It is an object of the present disclosure to provide a solution for monitoring physiological, cardiovascular and brain health parameters of users.

It is an object of the present disclosure to provide a solution for monitoring the blood flow of a user from region around neck of the user.

It is another object of the present solution to enable early detection of cardiovascular abnormalities and brain function abnormalities in users through continuous, non-invasive monitoring of physiological, cardiovascular and brain health parameters and any other such like parameters of the users.

It is another object of the present solution to provide a solution to optimize wearable device placement in any particular region/area of the body such as near jugular veins of the user.

It is yet another object of the present solution to provide a solution for enhanced hydration levels assessment using advanced far infrared (FIR) technology while minimizing inaccuracies caused by motion.

It is yet another object of the present disclosure to provide a solution for real-time monitoring of blood flow near neck without the need for invasive procedures.

It is yet another object of the present disclosure to provide a solution for early detection of various health issues/abnormalities such as cardiovascular abnormalities, brain edema, Jugular Venous Pressure, tricuspid regurgitation, tricuspid stenosis, pericardial effusion, or constrictive pericarditis, and such like health issues/abnormalities.

### BRIEF DESCRIPTION OF DRAWINGS

The accompanying drawings, which are incorporated herein, constitute a part of this disclosure. Components in the drawings are not necessarily to scale, emphasis instead being placed upon clearly illustrating the principles of the present disclosure. Some drawings may indicate the components using block diagrams and may not represent the internal circuitry of each component. It will be appreciated by those skilled in the art that disclosure of such drawings includes disclosure of electrical components or circuitry commonly used to implement such components. Although exemplary connections between sub-components have been shown in the accompanying drawings, it will be appreciated by those skilled in the art that other connections may also be possible, without departing from the scope of the disclosure. All sub-components within a component may be connected to each other, unless otherwise indicated.
FIG. 1 illustrates an exemplary block diagram of a device for monitoring physiological, cardiovascular and brain health parameters of users, in accordance with the exemplary embodiments of the present disclosure.
FIG. 1A illustrates an exemplary perspective view depicting placement of a device for monitoring physiological, cardiovascular and brain health parameters of users, in accordance with the exemplary embodiments of the present disclosure.
FIG. 2 illustrates an exemplary flow diagram of a method for monitoring physiological, cardiovascular and brain health parameters of users, in accordance with the exemplary embodiments of the present disclosure.

The foregoing shall be more apparent from the following more detailed description of the disclosure.

### DETAILED DESCRIPTION

In the following description, for the purposes of explanation, various specific details are set forth in order to provide a thorough understanding of embodiments of the present disclosure. It will be apparent, however, that embodiments of the present disclosure may be practiced without these specific details. Several features described hereafter may each be used independently of one another or with any combination of other features. An individual feature may not address any of the problems discussed above or might address only some of the problems discussed above.

The ensuing description provides exemplary embodiments only, and is not intended to limit the scope, applicability, or configuration of the disclosure. Rather, the ensuing description of the exemplary embodiments will provide those skilled in the art with an enabling description for implementing an exemplary embodiment. It should be understood that various changes may be made in the function and arrangement of elements without departing from the spirit and scope of the disclosure as set forth.

Specific details are given in the following description to provide a thorough understanding of the embodiments. However, it will be understood by one of ordinary skills in the art that the embodiments may be practiced without these specific details. For example, circuits, systems, processes, and other components may be shown as components in block diagram form in order not to obscure the embodiments in unnecessary detail.

Also, it is noted that individual embodiments may be described as a process which is depicted as a flowchart, a flow diagram, a data flow diagram, a structure diagram, or a block diagram. Although a flowchart may describe the operations as a sequential process, many of the operations may be performed in parallel or concurrently. In addition, the order of the operations may be re-arranged. A process is terminated when its operations are completed but could have additional steps not included in a figure.

The word "exemplary" and/or "demonstrative" is used herein to mean serving as an example, instance, or illustration. For the avoidance of doubt, the subject matter disclosed herein is not limited by such examples. In addition, any aspect or design described herein as "exemplary" and/or "demonstrative" is not necessarily to be construed as preferred or advantageous over other aspects or designs, nor is it meant to preclude equivalent exemplary structures and techniques known to those of ordinary skill in the art. Furthermore, to the extent that the terms "includes," "has," "contains," and other similar words are used in either the detailed description or the claims, such terms are intended to be inclusive-in a manner similar to the term "comprising" as an open transition word-without precluding any additional or other elements.

As used herein, an "apparatus" or an "electronic apparatus" or a "wearable apparatus" may refer to a set of devices used for the implementation of the technical solution provided by the present disclosure. Said apparatus may comprise one or more wearable devices that may either individually or collectively perform one or more functions for implementing the technical solutions of the present disclosure. The term "wearable apparatus" may refer to an apparatus that may be worn by a user of the wearable apparatus. Further, as used herein, the term "device" or "wearable device" or "electronic device" may further comprise one or more components or modules, which may be further used for implementing the technical solution of the present disclosure. For example, the wearable apparatus may be a smartwatch, and the wearable device may refer to a strap used for fastening the smartwatch on the hand of the user.

As used herein, a "module" or a "processing unit" includes one or more processors, wherein processor refers to any logic circuitry for processing instructions. A processor may be a general-purpose processor, a special purpose processor, a conventional processor, a digital signal processor, a plurality of microprocessors, one or more microprocessors in association with a DSP core, a controller, a microcontroller, Application Specific Integrated Circuits, Field Programmable Gate Array circuits, any other type of integrated circuits, etc. The processor may perform signal coding, data processing, input/output processing, and/or any other functionality that enables the working of the system according to the present disclosure. More specifically, the processor or processing unit is a hardware processor.

As used herein, "a user device" may be any electrical, electronic, and/or computing device or equipment, capable of implementing the features of the present disclosure. The user equipment/device may include, but is not limited to, a mobile phone, smart phone, laptop, a general-purpose computer, desktop, personal digital assistant, tablet computer, wearable device or any other computing device which is capable of implementing the features of the present disclosure. Also, the user device may contain at least one input means configured to receive an input from at least one of a transceiver unit, a processing unit, a storage unit, a detection unit and any other such unit(s) which are required to implement the features of the present disclosure.

As used herein, the "user device" and/or "wearable device" and/or "module" may comprise at least "storage unit" or "memory unit", wherein "storage unit" or "memory unit" refers to a machine or computer-readable medium including any mechanism for storing information in a form readable by a computer or similar machine. For example, a computer-readable medium includes read-only memory ("ROM"), random access memory ("RAM"), magnetic disk storage media, optical storage media, flash memory devices or other types of machine-accessible storage media. The storage unit stores at least the data that may be required by one or more units of the system to perform their respective functions.

As used herein, the expression "and/or" includes any single item from items or a combination of items associated with the items. For example, a group of A, B and/or C includes only A, only B, only C, a combination of A and B, a combination of B and C, a combination of A and C or a combination of A, B and C.

As used herein the expression "a set of' shall be interpreted as a collection of dataset or elements. The set may include a finite set with one element or more than one element. In an example, a set of elements may be made by an array of elements. For example, "a set of X" includes, {X}, {XX} and/or {X1, X2, X3}. Further, "the set of X" may also include an element Y that may or may not be related to X unless repugnant to the context thereof.

As used herein, the expression "one or more of' includes any single item in the list or a combination of items in the list. For example, one or more of A, B and C includes only A, only B, only C, a combination of A and B, a combination of B and C, a combination of A and C, a combination of A, B and C, a combination of multiple A and multiple B, a combination of multiple A, a single B and a single C, a combination of single A, multiple B and multiple C and any other such like combinations.

As used herein the expression "at least one of' shall be interpreted as an inclusive term that includes at least one of the succeeding elements, and shall also include multiple of such elements in different combinations. For example, the term "an exemplary parameter comprising at least one of A, B and C" may imply that the exemplary parameter may comprise only {A} or only {B} or only {C}, or {A, B, C} collectively, and may also comprise various combinations of A, B, and C (with or without any other element such as D), such as {A, B}, {B, C}, {C, A}, {C, D}, {D, A} and any other such like combinations. Further, the expression shall also be construed to be include multiple instances of such elements for example {A, A}, {A, A, D}, {A, B, C, A, B, C}, {A, B, C, A, B, C, D}, etc.

As discussed in the background section, the current known solutions have several shortcomings. The present disclosure aims to overcome the above-mentioned and other existing problems in this field of technology by providing a novel and inventive solution i.e., a device for monitoring physiological, cardiovascular and brain health parameters of users. Further, the device as disclosed in the present disclosure is configured to collect user data in real time from a target region such as a jugular vein and high blood flow location(s) of the user. Further, the novel and inventive solution of the present disclosure combines the user data collected by the different sensors and provides an enhanced solution for monitoring the physiological, the cardiovascular and the brain health parameters of the users. Further, the novel and inventive solution of the present disclosure is configured to filter the noise from the user data and compensate for the movement artifacts in the user data to generate accurate results related to monitored physiological, cardiovascular and brain health parameters of the users. Furthermore, the novel and inventive solution of present disclosure discloses an enhanced solution for early detection of abnormalities associated with the user from the collected user data by utilising advanced processing techniques.

Hereinafter, exemplary embodiments of the present disclosure will be described with reference to the accompanying drawings.

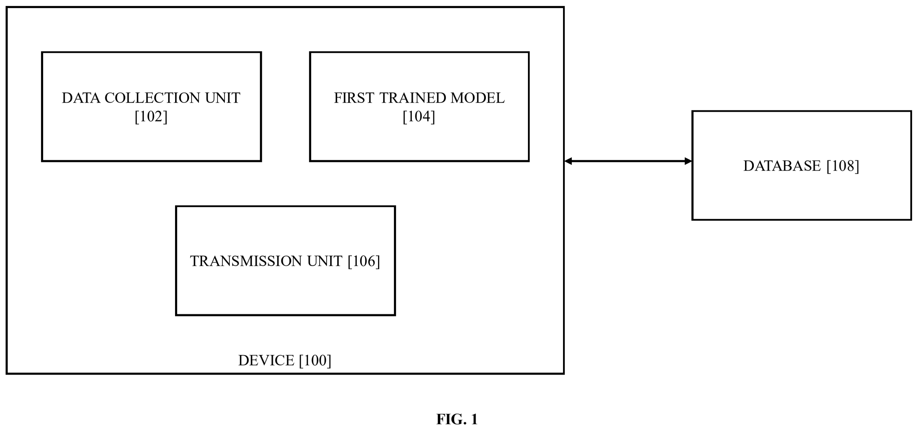

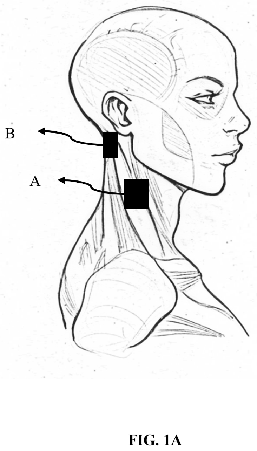

Referring to **FIG. 1** an exemplary block diagram of a device [100] for monitoring physiological, cardiovascular and brain health parameters of users, in accordance with the exemplary embodiments of the present disclosure is illustrated. The device [100] comprises at least one data collection unit [102], at least one first trained model [104] and at least one transmission unit [106]. All the units in the device [100] are communicatively coupled to each other in a manner as obvious to a person skilled in the art for implementing features of the present disclosure. Also, in FIG. 1 only a few units are shown, however, the device [100] may comprise multiple such units, as may be required to implement the features of the present disclosure. Further, in an embodiment, the device [100] may be connected to and/or in communication with a user device (may also be referred herein as a user equipment or a UE) to implement the features of the present disclosure. Furthermore, in another embodiment, the device [100] may be connected to and/or in communication with a server to implement the features of the present disclosure. Moreover, in an implementation the user device may comprise a database [108]. In another implementation, the server may comprise a database [108]. Further, the database [108] is communicatively coupled with the device [100] in a manner as obvious to a person skilled in the art for implementing features of the present disclosure.

Further referring to **FIG. 1A** an exemplary perspective view depicting placement of the device [100] for monitoring physiological, cardiovascular and brain health parameters of users, in accordance with the exemplary embodiments of the present disclosure is illustrated. Also, for ease of understanding FIG. 1 and FIG. 1A are explained in conjunction in the foregoing description for explanation of the technical solution as disclosed by the present disclosure. In one embodiment the device [100] may be affixed in a particular region/area such as region A as depicted in FIG. 1A, wherein the region A is a region around the neck area of the user for monitoring physiological, cardiovascular and brain health parameters of the user. In another embodiment of the present disclosure, as shown in the FIG. 1A, the device [100] may be affixed on the region B i.e., is a region around the ear area of the user for monitoring physiological, cardiovascular and brain health parameters of the user.

It is to be noted that the placement of the device [100] as shown in FIG. 1A i.e., in the region A i.e., the reign around neck area and in the region B i.e., the region around the ear area are only exemplary and in no manner intended to limit the scope of present disclosure. The device [100] may be affixed on any other particular region/area of the body of the user as appreciated by a person skilled in the art to implement the features of the present disclosure.

Also, in one embodiment of the present disclosure, the device [100] may be integrated into a neckband or a collar-type wearable device. In another embodiment of the present disclosure, the device [100] may be adapted for adhesive patches with disposable sensor modules.

It is to be noted that the abovementioned implementation describing the integration of the device [100] in different types of wearable devices is only exemplary and in no manner intended to limit the scope of the present disclosure. The device [100] may be used in any other form and/or integrated in any other type of wearable devices as appreciated by the person skilled in the art to implement the features of the present disclosure.

Referring again to FIG 1, in operation for monitoring the physiological, the cardiovascular and the brain health parameters of the user, the data collection unit [102] comprising at least a set of sensors, is configured to receive a set of user data associated with the user. In an implementation, the set of sensors comprises at least one of a Photoplethysmography (PPG) sensor, and a Far Infrared (FIR) sensor. The PPG sensor may refer to a sensor that is used for collecting information related to function of a cardiovascular organs of the user such as a heart rate of the user, a heart rate variability (HRV) of the user and an oxygen level (SpO2) in blood of the user. Further, the FIR sensor may refer to a sensor configured to monitor in real-time one or more physical attributes of the user such as a body temperature of the user and any other such like attribute. Further, the increase in the temperature of the user body may indicate that the hydration levels of the user are lower as compared to the normal hydration levels of the user. Further, the hydration levels refer to the measurement of the fluid levels (e.g., water level) in the user body. The measuring of the hydration levels involves assessing the body's fluid balance i.e., measuring the concentration of particles in body fluids of the user such as the blood and the urine and/or measuring the density of the body fluids, and any other such like parameters capable to indicate the hydration levels of the user. Further, the disturbance/disorder in the hydration level may significantly affect the temperature regulation of the user body. For example, when dehydrated the temperature of the body may rise as the ability of the body to regulate the body temperature is significantly impacted due to lower hydration levels (i.e., due to dehydration). However, it is to be noted that the abovementioned set of sensors are only exemplary and in no manner intended to limit the scope of the present disclosure and the set of sensors may include any other sensor(s) as appreciated by a person skilled in the art to implement the features of the present disclosure.

Further, the set of user data comprises at least one of a real time physiological health data, a real time cardiovascular health data and a real time brain health data. Further, in an implementation, the real time physiological health data may include data such as, but not limited to, blood oxygen levels of the user at a particular time, a respiratory rate of the user at a particular time, a body temperature of the user at a particular time and such like other data associated with the physiological health of the user collected at a particular time. Further, in an implementation, the real time cardiovascular health data may include data such as a heart rate of the user at a particular time, abnormal rhythms in the heartbeat of the user at a particular time, an Electrocardiogram (ECG) indicating electrical activity of the heart at a particular time and such like other data associated with the cardiovascular health of the user collected at a particular time. Furthermore, in an implementation, the real time brain health data include data such as data related to mental state of the user such as, but not limited to a parameter indicating stress level of the user at a particular time, a parameter indicating focus level of the user at a particular time, a parameter indicating a relaxation level of the user at a particular time and any other such like parameter indicating mental states of the user at a particular time.

Further, the set of user data is received, via the set of sensors, from a target body location of the user. In an exemplary embodiment of the present disclosure, the target body location may refer to a specific location on a body of the user for monitoring the set of user data in real time. The target body location is determined based on a set of body location determination rules. Further, the set of body location determination rules is based on at least one of a jugular vein location and a high blood flow location of the user. In an exemplary embodiment of the present disclosure, the set of body location determination rules may include rules such as, but not limited to, a high blood flow location determination rule, a cardiovascular system location determination rule, a rule for determination of a location with more perfusion, a rule for determination of a location with less movements and such like other body location determination rules to determine the target body location.

Further, in an implementation, the target body location may include such as, but not limited to, the jugular vein location in the body of the user and the high blood flow location in the body of the user. As referred herein the "jugular veins" refers to veins that are responsible for draining blood from the brain, the face and/or the neck of the user to the heart of the user. Also, the jugular veins are also located in the neck area of the user **(depicted as region A in the** **FIG. 1A****).** The region A in the FIG. 1A depicts the neck area of the user where the jugular vein are located. Further, in an implementation, the high blood flow location in the body of the user may refer to one or more locations having higher supply of blood as compared to other locations in the human body such as, the heart, the brain, the area behind the ears of the user **(depicted as region B in** **FIG. 1A****)** and such other body areas of the user. The region B is the FIG. 1A depicts the area behind the ears of the area, however, the same is not intended to limit the scope of the present disclosure.

It is to be noted that the abovementioned target body locations are only exemplary and in no manner intended to limit the scope of the present disclosure. The target body location may include any other location as appreciated by the person skilled in the art to implement the features of the present disclosure.

Further, the first trained model [104] is configured to integrate, one or more target user data from the set of user data based on one or more predefined data integration technique. In an implementation, the one or more target user data may refer to a specific health data related associated with the user. For example, for the cardiovascular health parameter the target user data may be the heart rate data and the HRV data of the user. Whereas for the brain health parameter, the target user data may be SpO2 data i.e., the amount of oxygen received by the brain of the user. Similarly, to measure other health parameters any other target user data as may be obvious to the person skilled in the art may be utilised by the device [100]. The first trained model [104] integrates the one or more target user data received from the set of sensors such as, but not limited to, the PPG sensor and the FIR sensor. Further, the integrated one or more target user data comprises combination of at least one of the heart rate, the HRV, the SpO2 and the hydration levels of the user.

Consider an example, to monitor the cardiovascular health of the user, the first trained model [104] may integrate the heart rate to measure the number of beats per minute and the HRV to measure the change in a time interval between each heartbeat. The combination of the heart rate and the HRV may provide an accurate data associated with the cardiovascular health of the user.

Further, the one or more predefined data integration technique is at least one of a signal processing technique and a data processing technique. In an implementation, the signal processing technique may refer to a technique that comprises analyzing the signals received by the device [100] to improve the accuracy of the one or more target user data for said monitoring of the physiological, cardiovascular and brain health parameters of the user. Further, in an implementation, the data processing technique may refer to a technique in which the data received in a raw format may be transformed into a target format i.e., a format required for said monitoring of the physiological, cardiovascular and brain health parameters of the user.

Considering an example, to monitor the hydration levels of the user at time T1, the first trained model [104] may utilize the signal processing technique to analyse the heart rate of the user at said time to extract an indicator from the set of user that indicates the user is resting, the user is moving, the user is running and such like other information. Further, let's say, based on the analysis the first trained model [104] determines that the user is running at time T1. Further, the first trained model [104] may utilize the signal processing technique to analyse the temperature of the user body at different time durations and during different user activities. The first trained model [104] may utilize the data processing technique to combine the heart rate of the user at time T1 and the temperature of the user body at time T1. The combination of the heart rate at time T1 and the temperature at time T1 may provide an accurate data associated with the hydration levels of the user at time T1 associated with the current state of that user i.e., running.

Further, to integrate the one or more target user data, the first trained model [104] is configured to fetch, from the database [108], a set of historical user data associated with the user. In an exemplary embodiment of the of the present disclosure, the database [108] may be at least one of a cloud storage based database and a storage of the user equipment (UE) connected with the device [100]. The set of user data received in the real time may be stored in at least one of the cloud storage and the storage of the UE connected to the device [100] to facilitate continuous monitoring of said physiological, cardiovascular and brain health parameters of the user and provide a real-time analysis of overall health to the user. Further, to facilitate the continuous monitoring, the set of real time user data collected over a period of time such as past 1 month, is stored in the database [108] to generate the set of historical user data. Further, the target user data can be integrated based on the set of real time user data and the set of historical user data. Further, the set of historical user data comprises at least one of a set of past physiological data, a set of past cardiovascular data and a set of past brain health data associated with the user. In an exemplary embodiment of the present disclosure, the set of historical user data may refer to the set of user data that are received in the past over a predefined period time and are stored in the database [108].

Further, the first trained model [104] is configured to determine, the one or more target user data based on comparing the set of user data and the set of historical user data. In an implementation, the first trained model [104] compares the set of user data and the set of historical user data to determine the one or more target user data.

Consider an example, to monitor the cardiovascular health of the user, the first trained model [104] first determines the one or more target user data related to the cardiovascular health of the user. Further, to determine the one or more target user data the first trained model [104] compares the set of real time user data with the set of historical user data. According to the set of historical user data to monitor the cardiovascular health of the user the one or more target user data may be the heart rate data and the HRV data associated with the user. Therefore, the first trained model [104] may determine the heart rate and the HRV data as the target user data to monitor the cardiovascular health of the user.

Furthermore, the first trained model [104] is configured to generate, the integrated one or more target user data based on the determined one or more target user data, and at least one predefined data integration technique from the one or more predefined data integration technique. Once the one or more target user data is determined the first trained model [104] is configured to integrate the determined one or more target user data to generate the integrated one or more target user data.

Further, the first trained model [104] is configured to generate, an enhanced dataset based on the integrated one or more target user data. In an implementation, the set of user data, the set of historical user data and the integrated one or more target user data may comprise at least one of a relevant dataset and an ancillary dataset. Further, the relevant dataset may include a set of data that is useful and/or relevant for monitoring the physiological, cardiovascular and brain health parameters of the user. Whereas the ancillary dataset may include a set of additional data associated with the physiological, cardiovascular and brain health parameters of the user that is received along with the relevant dataset. Furthermore, in the implementation the enhanced data set may refer to the relevant dataset associated with the physiological, cardiovascular and brain health parameters of the user.

Further, the first trained model [104] is configured to detect, a noise parameter associated with the integrated one or more target user data based on analyzing one or more of the set of user data, the set of historical user data and the integrated one or more target user data. In an implementation, the noise parameter associated with the integrated one or more target user data may refer to the ancillary dataset. Furthermore, in an implementation the noise parameters may comprise irrelevant data associated with the user that may interfere in accurately monitoring the physiological, cardiovascular and brain health parameters of the user. Also, the noise parameter is generated based on at least one of a real time movement associated with the user and an activity performed by the user. Additionally, the noise parameter is detected from the set of user data, the set of historical user data and the integrated one or more target user data based on a predefined noise detection technique. In an exemplary embodiment of the present disclosure, the predefined noise detection technique may refer to a technique that may be used to identify and differentiate the relevant dataset from the ancillary dataset to improve the accuracy of the set of user data, the set of historical user data and the integrated one or more target user data. Moreover, the first trained model [104] is configured to filter, the noise parameter from the one or more target user data to generate the enhanced dataset. Once the relevant dataset are identified and differentiated from the ancillary dataset based on the predefined noise detection technique, the first trained model [104] removes (i.e., filters) the ancillary dataset (i.e., the noise parameter) from the set of user data, the set of historical user data and the integrated one or more target user data to generate the enhanced dataset (i.e., relevant dataset). For example, for accurately monitoring the heart rate of the user the first trained model [104] may utilize the predefined noise detection technique to filter/remove all the noise from the monitored heart rate that may have been added due to various movement artifacts of the user.

Once the enhanced dataset is generated, the first trained model [104] is further configured to detect, one or more abnormalities associated with the user based on at least the enhanced dataset. The one or more abnormalities is at least one of one or more physiological abnormalities, one or more cardiovascular abnormalities and one or more brain functions abnormalities associated with the user. In an exemplary embodiment of the present disclosure, the one or more physiological abnormalities may refer to one or more disorders in the normal/proper functioning of the body of the user, that may be caused due malfunctioning of the organs in the body of the user. Further, the one or more physiological abnormalities may affect the normal functioning of the various systems of the user such as a respiratory system, a blood circulatory system, a nervous system and such like other systems. Further, in an exemplary embodiment of the present disclosure, the one or more cardiovascular abnormality may refer to a deviation in the normal functioning of a cardiovascular system of the user such as the heart of the user and any other such like systems. Furthermore, exemplary embodiment of the present disclosure, the one or more brain function abnormality may refer to one or more disorders that may disrupt the normal functioning of the brain of the user. Further, said one or more disorders may affect the ability of the user to think, feel, and such like other abilities of the user. Further, said one or more disorders in the functioning of the brain includes disorders such as depression, anxiety and such like other disorders in the brain of the user.

Also, the first trained model [104] is configured to fetch, of a predefined time period, a set of historical user data associated with the user. In an implementation, to detect the one or more abnormalities, the first trained model [104] compares the set of real time user data with the set of historical user data of the predefined time period to identify the deviation from the normal functioning of the user body. Also, the predefined time period may include any fixed duration of time such as 7 days, 1 month, etc, as appreciated by a person skilled in the art to implement the features of the present disclosure. Consider an example, the device [100] detects a sudden increase in the heartbeat of the user at a particular time. The first trained model [104] will fetch the historical data of, say, 7 days from the database [108] to compare the real time heartbeat of the user and determine if a same pattern has been detected in the past 7 days also. In an event, based on the comparison, if the same pattern has been detected in the past 7 days also, the first trained model [104] will determine that there is no abnormality in the user data (i.e., there is no deviation in the functioning of the user body from the normal functioning of the user body). Whereas in another event if the same pattern has not been detected on any day from the past 7 days, the first trained model [104] will determine that there may be an abnormality associated with the user based on the detected deviation in the real time heartbeat in comparison to the past data associated with the heartbeat of the user.

Further, the first trained model [104] is configured to determine, a key health parameter associated with the user based on comparing the set of user data and the set of historical user data, wherein the key health parameter indicates the one or more abnormalities. In an implementation, the key health parameters may refer to measurement of the health parameters that deviates from the normal measurement of the health parameters (including the physiological, the cardiovascular and the brain health parameters). Further, said deviation in the measurement of the health parameters may indicate the one or more abnormalities in the user body. Furthermore, the key health parameter is determined in an event a value associated with one or more parameters of the enhanced dataset is above a predefined threshold value associated with said one or more parameters of the enhanced dataset.

Consider an example, the set of historical user data of, say, 7 days indicates that while running or jogging the heart rate of the user ranges between 150-170 bpm (beats per minute) and while resting the heart rate of the user ranges between 68-75 bpm. However, the set of real time user data detected the heart rate of say, 160 bpm while the user is resting which is above the predefined threshold value. Based on the comparison of the set of real time user data with the set of historical user data of 7 days, the first trained model [104] determines the key health parameter i.e., the deviation in the measurement of the heart rate of the user from the normal measurement of the heart rate of the user, when the user is resting, thereby indicating the abnormality in the cardiovascular health parameter of the user.

Moreover, the transmission unit [106] is configured to transmit, on a user device, a set of notification associated with the detected one or more abnormalities. In an exemplary embodiment of the present disclosure, the set of notification may include the key health parameter indicating the one or more abnormalities.

Continuing the above example, wherein the device [100] determines that the user is resting based on the predefined noise detection technique. The device [100] filters the set of real-time user data, such as body temperature and/or any movement artifacts, as ancillary data from the relevant data, such as heart rate. Next, by comparing the filtered set of real-time user data with the set of historical user data, the device [100] confirms that the user is indeed resting.

According to the historical user data, when the user is resting, the body temperature typically ranges between 36-38 degrees Celsius, the heart rate ranges between 70-75 bpm, and movement artifacts are very low. Upon filtering the set of real-time user data and comparing it with the set of historical data, the device [100] finds that both the body temperature and movement artifacts are consistent with values associated with resting from the set of historical data. However, the heart rate has increased to 160 bpm, indicating that the user's heart rate is abnormally high while resting.

In that event the transmission unit [106] may then send a notification to the user device, alerting the user to the abnormal heart rate during rest, specifically, that the heart rate has suddenly increased to 160 bpm while the user is resting. This enables early detection of a potential cardiovascular abnormality. Based on this notification, the user may take one or more appropriate precautionary measures.

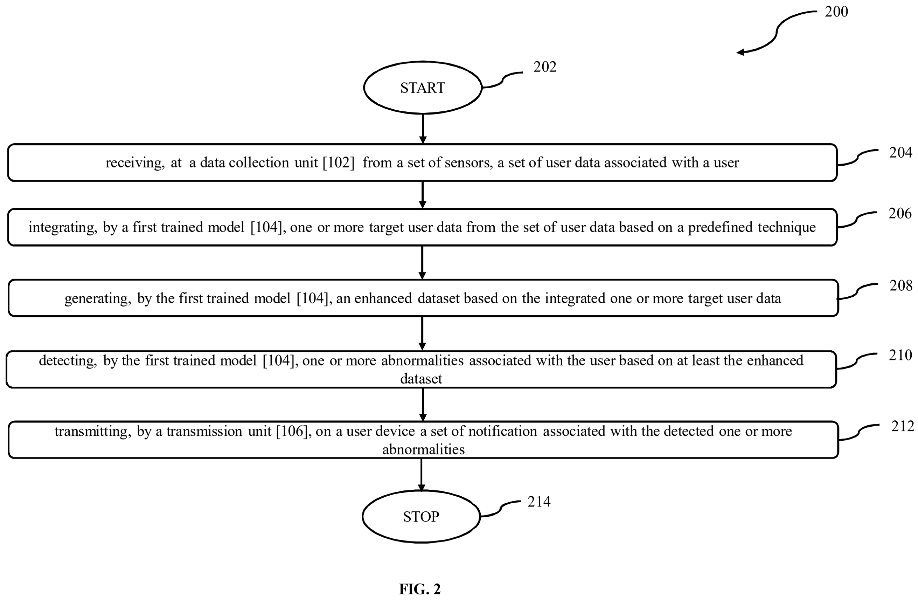

Referring to **FIG. 2** an exemplary flow diagram of a method [200] for monitoring physiological, cardiovascular and brain health parameters of users, in accordance with the exemplary embodiments of the present disclosure is illustrated. In an implementation, the method [200] is performed by the device [100]. The method [200] starts at step [202].

At step **[204],** the method [200] comprises receiving, at a data collection unit [102] from a set of sensors, a set of user data associated with a user. In an implementation, the set of sensors comprises at least one of a Photoplethysmography (PPG) sensor, and a Far Infrared (FIR) sensor. The PPG sensor may refer to a sensor that is used for collecting information related to function of a cardiovascular organs of the user such as heart rate of the user, heart rate variability (HRV) of the user and an oxygen level (SpO2) in blood of the user. Further, the FIR sensor may refer to a sensor configured to monitor in real time one or more physical attributes of the user such as a body temperature of the user and any other such like attribute. Further, the increase in the temperature of the user body may indicate that the hydration levels of the user are lower as compared to the normal hydration levels of the user. Further, the hydration levels refer to the measurement of the fluid levels (e.g., water level) in the user body. The measuring of the hydration levels involves assessing the body's fluid balance i.e., measuring the concentration of particles in body fluids of the user such as the blood and the urine, and/or measuring the density of the body fluid, and any other such like parameters capable to indicate the hydration levels of the user.. Further, the disturbance/disorder in the hydration level may significantly affect the temperature regulation of the user body. For example, when dehydrated the temperature of the body may rise as the ability of the body to regulate the body temperature is significantly impacted due to lower hydration levels (i.e., due to dehydration). However, it is to be noted that the abovementioned set of sensors are only exemplary and in no manner intended to limit the scope of the present disclosure. The set of sensors may include any other sensor(s) as appreciated by a person skilled in the art to implement the features of the present disclosure.

Further, the set of user data comprises at least one of a real time physiological health data, a real time cardiovascular health data and a real time brain health data. Further, in an implementation, the real time physiological health data may include data such as, but not limited to, blood oxygen levels of the user at a particular time, a respiratory rate of the user at a particular time, a body temperature of the user at a particular time and such like other data associated with the physiological health of the user collected at a particular time. Further, in an implementation, the real time cardiovascular health data may include data such as a heart rate of the user at a particular time, abnormal rhythms in the heartbeat of the user at a particular time, an Electrocardiogram (ECG) indicating electrical activity of the heart at a particular time and such like other data associated with the cardiovascular health of the user collected at a particular time. Furthermore, in an implementation, the real time brain health data include data such as data related to mental state of the user such as, but not limited to a parameter indicating stress level of the user at a particular time, a parameter indicating focus level of the user at a particular time, a parameter indicating a relaxation level of the user at a particular time and any other such like parameter indicating mental states of the user at a particular time.

Further, the set of user data is received, via the set of sensors, from a target body location of the user. In an exemplary embodiment of the present disclosure, the target body location may refer to a specific location on a body of the user for monitoring the set of user data in real time. The target body location is determined based on a set of body location determination rules. Further, the set of body location determination rules is based on at least one of a jugular vein location and a high blood flow location of the user. In an exemplary embodiment of the present disclosure, the set of body location determination rules may include rules such as, but not limited to, a high blood flow location determination rule, a cardiovascular system location determination rule, a rule for determination of a location with more perfusion, a rule for determination of a location with less movements and such like other body location determination rules to determine the target body location.

Further, in an implementation, the target body location may include such as, but not limited to, the jugular vein location in the body of the user and the high blood flow location in the body of the user. As referred herein the "jugular veins" refers to veins that are responsible for draining blood from the brain, the face and/or the neck of the user to the heart of the user. Also, the jugular veins are also located in the neck area of the user **(depicted as region A in the** **FIG. 1A****).** The region A in the FIG. 1A depicts the neck area of the user where the jugular vein are located. Further, in an implementation, the high blood flow location in the body of the user may refer to one or more locations having higher supply of blood as compared to other locations in the human body such as, the heart, the brain, the area behind the ears of the user **(depicted as region B in** **FIG. 1A****)** and such other body areas of the user. The region B is the FIG. 1A depicts the area behind the ears of the area, however, the same is not intended to limit the scope of the present disclosure.

It is to be noted that the abovementioned target body locations are only exemplary and in no manner intended to limit the scope of the present disclosure. The target body location may include any other location as appreciated by the person skilled in the art to implement the features of the present disclosure.

Next, at step **[206],** the method [200] comprises integrating, by a first trained model [104], one or more target user data from the set of user data based on the one or more predefined data integration technique. In an implementation, the one or more target user data may refer to a specific health data associated with the user. For example, for the cardiovascular health parameter the target user data may be the heart rate data and the HRV data of the user. Whereas for the brain health parameter the target user data may be SpO2 data i.e., the amount of oxygen received by the brain of the user. Similarly, to measure other health parameters any other target user data as may be obvious to the person skilled in the art may be utilised by the device [100]. The first trained model [104] integrates the one or more target user data received from the set of sensors such as, but not limited to, the PPG sensor and the FIR sensor. Further, the integrated one or more target user data comprises combination of at least one of the heart rate, the HRV, the SpO2 and the hydration levels of the user.

Consider an example, to monitor the cardiovascular health of the user, the first trained model [104] may integrate the heart rate to measure the number of beats per minute and the HRV to measure the change in a time interval between each heartbeat. The combination of the heart rate and the HRV may provide an accurate data associated with the cardiovascular health of the user.

Further, the one or more predefined data integration technique is at least one of a signal processing technique and a data processing technique. In an implementation, the signal processing technique may refer to a technique that comprises analyzing the signals received by the device [100] to improve the accuracy of the one or more target user data for said monitoring of the physiological, cardiovascular and brain health parameters of the user. Further, in an implementation, the data processing technique may refer to a technique in which the data received in a raw format may be transformed into a target format i.e., the format required for said monitoring of the physiological, cardiovascular and brain health parameters of the user.

Considering an example, to monitor the hydration levels of the user at time T1, the first trained model [104] may utilize the signal processing technique to analyse the heart rate of the user at said time to extract indicator from the set of user data that indicates the user is resting, the user is moving, the user is running and such like other information. Further, let's say, based on the analysis the first trained model [104] determines that the user is running at time T1. Further, the first trained model [104] may utilize the signal processing technique to analyse the temperature of the user body at different time durations and during different user activities. The first trained model [104] may utilize the data processing technique to combine the heart rate of the user at time T1 and the temperature of the user body at time T1. The combination of the heart rate at time T1 and the temperature at time T1 may provide an accurate data associated with the hydration levels of the user at time T1 associated with the current state of that user i.e., running.

Further, the method [200] comprises, fetching, by the first trained model [104] from a database [108], a set of historical user data associated with the user. In an exemplary embodiment of the of the present disclosure, the database [108] may be at least one of a cloud storage based database and a storage of the user equipment (UE) connected with the device [100]. The set of user data received in the real time may be stored in at least one of the cloud storage and the storage of the UE connected to the device [100] to facilitate continuous monitoring of said physiological, cardiovascular and brain health parameters of the user and provide a real-time analysis of overall health to the user. Further, to facilitate the continuous monitoring, the set of real time user data collected over a period of time such as past 1 month, is stored in the database [108] to generate the set of historical user data. Further, the target user data can be integrated based on the set of real time user data and the set of historical user data. Further, the set of historical user data comprises at least one of a set of past physiological data, a set of past cardiovascular data and a set of past brain health data associated with the user. In an exemplary embodiment of the present disclosure, the set of historical user data may refer to the set of user data that are received in the past over a predefined period time and are stored in the database [108].

Further, the method [200] comprises determining, by the first trained model [104], the one or more target user data based on comparing the set of user data and the set of historical user data. In an implementation, the first trained model [104] compares the set of user data and the set of historical user data to determine the one or more target user data.

Consider an example, to monitor the cardiovascular health of the user, the first trained model [104] first determines the one or more target user data related to the cardiovascular health of the user. Further, to determine the one or more target user data the first trained model [104] compares the set of real time user data with the set of historical user data. According to the set of historical user data to monitor the cardiovascular health of the user the one or more target user data may be the heart rate data and the HRV data associated with the user. Therefore, the first trained model [104] may determine the heart rate and the HRV data as the target user data to monitor the cardiovascular health of the user.

Furthermore, the method [200] comprises, generating, by the first trained model [104], the integrated one or more target user data based on the determined one or more target user data, and at least one predefined data integration technique from the one or more predefined data integration technique. Once the one or more target user data is determined the first trained model [104] is configured to integrate the determined one or more target user data to generate the integrated one or more target user data.

Further, at step **[208],** the method [200] comprises generating, by the first trained model [104], an enhanced dataset based on the integrated one or more target user data. In an implementation, the set of user data, the set of historical user data and the integrated one or more target user data may comprise at least one of a relevant dataset and an ancillary dataset. Further, the relevant dataset may include a set of data that is useful and/or relevant for monitoring the physiological, cardiovascular and brain health parameters of the user. Whereas the ancillary dataset may include a set of additional data associated with the physiological, cardiovascular and brain health parameters of the user that is received along with the relevant dataset. Furthermore, in the implementation the enhanced data set may refer to the relevant dataset associated with the physiological, cardiovascular and brain health parameters of the user.

The method [200] further comprises detecting, by the first trained model [104], a noise parameter associated with the integrated one or more target user data based on analyzing one or more of the set of user data, the set of historical user data and the integrated one or more target user data. In an implementation, the noise parameter associated with the integrated one or more target user data may refer to the ancillary dataset. Furthermore, in an implementation the noise parameters may comprise irrelevant data associated with the user that may interfere in accurately monitoring the physiological, cardiovascular and brain health parameters of the user. Also, the noise parameter is generated based on at least one of a real time movement associated with the user and an activity performed by the user. Additionally, the noise parameter is detected from the set of user data, the set of historical user data and the integrated one or more target user data based on a predefined noise detection technique.

Further, the method [200] comprises filtering, by the first trained model [104], the noise parameter from the one or more target user data to generate the enhanced dataset. Once the relevant dataset are identified and differentiated from the ancillary dataset based on the predefined noise detection technique, the first trained model [104] removes (i.e., filters) the ancillary dataset (i.e., the noise parameter) from the set of user data, the set of historical user data and the integrated one or more target user data to generate the enhanced dataset (i.e., relevant dataset). For example, for accurately monitoring the heart rate of the user the first trained model [104] may utilize the predefined noise detection technique to filter/remove all the noise from the monitored heart rate that may have been added due to various movement artifacts of the user.

Further, at step **[210],** the method [200] comprises detecting, by the first trained model [104], one or more abnormalities associated with the user based on at least the enhanced dataset. The one or more abnormalities is at least one of one or more physiological abnormalities, one or more cardiovascular abnormalities and one or more brain functions abnormalities associated with the user. In an exemplary embodiment of the present disclosure, the one or more physiological abnormalities may refer to one or more disorders in the normal/proper functioning of the body of the user, that may be caused due malfunctioning of the organs in the body of the user. Further, the one or more physiological abnormalities may affect the normal functioning of the various systems of the user such as a respiratory system, a blood circulatory system, a nervous system and such like other systems.. Further, in an exemplary embodiment of the present disclosure, the one or more cardiovascular abnormality may refer to a deviation in the normal functioning of a cardiovascular system of the user such as heart of the user and any other such like systems. Furthermore, exemplary embodiment of the present disclosure, the one or more brain function disorders that may disrupt the normal functioning of the brain of the user. Further, said one or more disorders may affect the ability of the user to think, feel, and such like other abilities of the user. Further, said one or more disorders in the functioning of the brain includes disorders such as depression, anxiety and such like other disorders in the brain of the user.

Also, the method [200] comprises fetching, by the first trained model [104], a set of historical user data, of a predefined period of time, associated with the user of a predefined time period. In an implementation, to detect the one or more abnormalities the first trained model [104] compares the set of real time user data with the set of historical user data of the predefined time period to identify the deviation from the normal functioning of the user body. Also, the predefined time period may include fixed duration of time such as 7 days, 1 month, etc, as appreciated by a person skilled in the art to implement the features of the present disclosure. Consider an example, the device [100] detects a sudden increase in the heartbeat of the user at a particular time. The first trained model [104] will fetch the historical data of, say, 7 days from the database [108] to compare the real time heartbeat of the user and determine if a same pattern has been detected in the past 7 days also. In an event, based on the comparison, if the same pattern has been detected in the past 7 days also, the first trained model [104] will determine that there is no abnormality in the user data (i.e., there is no deviation in the functioning of the user body from the normal functioning of the user body). Whereas in another event if the same pattern has not been detected on any day from the past 7 days, the first trained model [104] will determine that there may be an abnormality associated with the user based on the detected deviation in the real time heartbeat in comparison to the past data associated with the heartbeat of the user.

Further, the method [200] comprises determining, by the first trained model [104], a key health parameter associated with the user based on comparing the set of user data and the set of historical user data, wherein the key health parameter indicates the one or more abnormalities. In an implementation, the key health parameters may refer to measurement of the health parameters that deviates from the normal measurement of the health parameters (including the physiological, the cardiovascular and the brain health parameters). Further, said deviation in the measurement of the health parameters may indicate the one or more abnormalities in the user body. Furthermore, the key health parameter is determined in an event a value associated with one or more parameters of the enhanced dataset is above a predefined threshold value associated with said one or more parameters of the enhanced dataset.

Consider an example, the set of historical user data of, say, 7 days indicates that while running or jogging the heart rate of the user ranges between 150-170 bpm (beats per minute) and while resting the heart rate of the user ranges between 68-75 bpm. However, the set of real time user data detected the heart rate of say, 160 bpm while the user is resting which is above the predefined threshold value. Based on the comparison of the set of real time user data with the set of historical user data of 7 days, the first trained model [104] determines the key health parameter i.e., the deviation in the measurement of the heart rate of the user from the normal measurement of the heart rate of the user, when the user is resting, thereby indicating the abnormality in the cardiovascular health parameter of the user.

Furthermore, at step **[212],** the method [200] comprises transmitting, by a transmission unit [106], on a user device a set of notification associated with the detected one or more abnormalities. In an exemplary embodiment of the present disclosure, the set of notification may include the key health parameter indicating the one or more abnormalities.

Continuing the above example, wherein the device [100] determines that the user is resting based on the predefined noise detection technique. The device [100] filters the set of real-time user data, such as body temperature and/or any movement artifacts, as ancillary data from the relevant data, such as heart rate. Next, by comparing the filtered set of real-time user data with the set of historical user data, the device [100] confirms that the user is indeed resting.

According to the historical user data, when the user is resting, the body temperature typically ranges between 36-38 degrees Celsius, the heart rate ranges between 70-75 bpm, and movement artifacts are very low. Upon filtering the set of real-time user data and comparing it with the set of historical data, the device [100] finds that both the body temperature and movement artifacts are consistent with values associated with resting from the set of historical data. However, the heart rate has increased to 160 bpm, indicating that the user's heart rate is abnormally high while resting.

In that event the transmission unit [106] may then send a notification to the user device, alerting the user to the abnormal heart rate during rest, specifically, that the heart rate has suddenly increased to 160 bpm while the user is resting. This enables early detection of a potential cardiovascular abnormality. Based on this notification, the user may take one or more appropriate precautionary measures. Thereafter, at step **[214],** the method [200] terminates.

As evident from the above, the present disclosure provides a technically advanced solution to monitor the physiological, cardiovascular and brain health parameters of the user. The present disclosure provides a technically advanced solution that efficiently and accurately monitors the physiological, cardiovascular and brain health parameters of the user in real time by intelligently identifying and integrating the data related to a particular health parameter received from various sensors. Further, the technically advanced solution of the present disclosure not only efficiently and accurately monitors the health parameters of the user but also facilitates in early detection of one or more abnormalities associated with said particular organ/system of the user. Furthermore, the technically advanced solution of the present invention provides the device that is optimized to be placed in other region/area such as in a jugular vein area and a high blood flow area unlike the existing wrist and finger worn devices to improve the accuracy in monitoring the physiological, cardiovascular and brain health parameters of the user.

While the present invention has been described with reference to certain preferred embodiments and examples thereof, other embodiments, equivalents and modifications are possible and are also encompassed by the scope of the present disclosure.

## Claims

1. A device for monitoring physiological, cardiovascular and brain health parameters of a user, the device comprises:
- a data collection unit [102] comprising at least a set of sensors, wherein the data collection unit [102] is configured to receive a set of user data associated with a user;
- a first trained model [104] connected to at least the data collection unit [102], wherein the first trained model [104] is configured to:
o integrate, one or more target user data from the set of user data based on one or more predefined data integration technique;
o generate, an enhanced dataset based on the integrated one or more target user data;
o detect, one or more abnormalities associated with the user based on at least the enhanced dataset; and
- a transmission unit [106] connected to at least the first trained model [104], wherein the transmission unit [106] is configured to transmit, on a user device, a set of notification associated with the detected one or more abnormalities.

2. The device as claimed in claim 1 is configured to receive via the set of sensors, the set of user data from a target body location of the user, wherein the set of sensors comprises at least one of a Photoplethysmography (PPG) sensor, and a Far Infrared (FIR) sensor, and wherein the set of user data comprises at least one of a real time physiological health data, a real time cardiovascular health data and a real time brain health data.

3. The device as claimed in claim 2, wherein the target body location is determined based on a set of body location determination rules, and wherein the set of body location determination rules is based on at least one of a jugular vein location and a high blood flow location of the user.

4. The device as claimed in any of claims 1 to 3, wherein the first trained model [104] is further configured to:
- fetch, from a database [108], a set of historical user data associated with the user, wherein the set of historical user data comprises at least one of a set of past physiological data, a set of past cardiovascular data and a set of past brain health data associated with the user;
- determine, the one or more target user data based on comparing the set of user data and the set of historical user data; and
- generate, the integrated one or more target user data based on the determined one or more target user data, and at least one predefined data integration technique from the one or more predefined data integration technique, wherein the one or more predefined data integration technique is at least one of a signal processing technique and a data processing technique.

5. The device as claimed in any of claims 1 to 4, wherein the first trained model [104] is further configured to:
- detect, a noise parameter associated with the integrated one or more target user data based on analyzing one or more of the set of user data, the set of historical user data and the integrated one or more target user data, wherein the noise parameter is generated based on at least one of a real time movement associated with the user and an activity performed by the user,
and wherein the noise parameter is detected from the set of user data, the set of historical user data and the integrated one or more target user data based on a predefined noise detection technique; and
- filter, the noise parameter from the one or more target user data to generate the enhanced dataset.

6. The device as claimed in any of claims 1 to 5, wherein the one or more abnormalities is at least one of one or more physiological abnormalities, one or more cardiovascular abnormalities and one or more brain functions abnormalities associated with the user.

7. The device as claimed in any of claims 1 to 6, wherein the first trained model [104] is further configured to:
- fetch, a set of historical user data associated with the user of a predefined time period; and
- determine, a key health parameter associated with the user based on comparing the set of user data and the set of historical user data, wherein the key health parameter indicates the one or more abnormalities, and wherein the key health parameter is determined in an event a value associated with one or more parameters of the enhanced dataset is above a predefined threshold value associated with said one or more parameters of the enhanced dataset.

8. A method for monitoring physiological, cardiovascular and brain health parameters of a user, the method comprises:
- receiving, at a data collection unit [102] from a set of sensors, a set of user data associated with a user;
- integrating, by a first trained model [104], one or more target user data from the set of user data based on one or more predefined data integration technique;
- generating, by the first trained model [104], an enhanced dataset based on the integrated one or more target user data;
- detecting, by the first trained model [104], one or more abnormalities associated with the user based on at least the enhanced dataset; and
- transmitting, by a transmission unit [106], on a user device a set of notification associated with the detected one or more abnormalities.

9. The method as claimed in claim 8, wherein the method further comprises receiving, via the set of sensors from a target body location of the user, the set of user data, wherein the set of sensors comprises at least one of a Photoplethysmography (PPG) sensor, and a Far Infrared (FIR) sensor, and wherein the set of user data comprises at least one of a real time physiological health data, a real time cardiovascular health data and a real time brain health data.

10. The method as claimed in claim 9, wherein the target body location is determined based on a set of body location determination rules, and wherein the set of body location determination rules is based at least one of a jugular vein location and a high blood flow location of the user.

11. The method as claimed in any of claims 8 to 10, wherein the method further comprises:
- fetching, by the first trained model [104] from a database [108], a set of historical user data associated with the user, wherein the set of historical user data comprises at least one of a set of past physiological data, a set of past cardiovascular data and a set of past brain health data associated with the user;
- determining, by the first trained model [104], the one or more target user data based on comparing the set of user data and the set of historical user data; and
- generating, by the first trained model [104], the integrated one or more target user data based on the one or more target user data, and at least one predefined data integration technique from the one or more predefined data integration technique, wherein the one or more predefined data integration technique is at least one of a signal processing technique and a data processing technique.

12. The method as claimed in any of claims 8 to 11, wherein the method further comprises:
- detecting, by the first trained model [104], a noise parameter associated with the integrated one or more target user data based on analyzing one or more of the set of user data, the set of historical user data and the integrated one or more target user data, wherein the noise parameter is generated based on at least one of a real time movement associated with the user and an activity performed by the user,
and wherein the noise parameter is detected from the set of user data, the set of historical user data and the integrated one or more target user data based on a predefined noise detection technique; and
- filtering, by the first trained model [104], the noise parameter from the one or more target user data to generate the enhanced dataset.

13. The method as claimed in any of claims 8 to 12, wherein the one or more abnormalities is at least one of one or more physiological abnormalities, one or more cardiovascular abnormalities and one or more brain functions abnormalities associated with the user.

14. The method as claimed in any of claims 8 to 13, wherein the method further comprises:
- fetching, by the first trained model [104], a set of historical user data associated with the user of a predefined time period; and
- determining, by the first trained model [104], a key health parameter associated with the user based on comparing the set of user data and the set of historical user data, wherein the key health parameter indicates the one or more abnormalities, and wherein the key health parameter is determined in an event a value associated with one or more parameters of the enhanced dataset is above a predefined threshold value associated with said one or more parameters of the enhanced dataset.
